# EUROPÄISCHE PATENTSCHRIFT

(11) **EP 1 967 217 B1**
(45) Veröffentlichungstag und Bekanntmachung des Hinweises auf die Patenterteilung: **26.10.2011**
(21) Anmeldenummer: 08004219.5
(22) Anmeldetag: 07.03.2008
(51) Int. Cl.: A61L 15/18, A61L 15/22, A61L 15/44, A61L 17/00, A61L 17/10, A61L 27/44, A61L 27/54

(54) **Antimikrobielles medizintechnisches Produkt, Verfahren zu seiner Herstellung und Verwendung**
Antimicrobial medical product, method for manufacture and application thereof
Produit médical antimicrobien, son procédé de fabrication et utilisation

(30) Priorität: 09.03.2007 DE 102007012253
(43) Veröffentlichungstag der Anmeldung: 10.09.2008
(73) Patentinhaber: Aesculap AG, 78532 Tuttlingen (DE); Albert-Ludwigs-Universität Freiburg, 79085 Freiburg (DE)
(72) Erfinder: Odermatt, Erich, 8200 Schaffhausen (CH); Berndt, Ingo, 78532 Tuttlingen (DE); Tiller, Jörg, 79110 Freiburg (DE); Ho, Chau Hon, 79106 Freiburg (DE)
(74) Vertreter: Patentanwälte Ruff, Wilhelm, Beier, Dauster & Partner

(56) Entgegenhaltungen:
- WO-A2-2004/056404
- DE-A1- 19 921 894
- DE-A1-102005 044 360
- DE-A1-102006 011 217
- CYRIL AYMONIER, ULF SCHLOTTERBECK, JOERG C. TILLER, STEFAN MECKING AND AL.: "Hybrids of silver nanoparticles with amphiphilic hyperbranched macromolecules exhibiting antimicrobial properties" CHEMICAL COMMUNICATION, 19. November 2002 (2002-11-19), Seiten 3018-3019, XP002543442

## Beschreibung

Die Erfindung betrifft ein medizintechnisches Produkt mit einer antimikrobiellen Ausstattung, ein Verfahren zu dessen Herstellung sowie die Verwendung der antimikrobiellen Ausstattung als Biozid bei medizintechnischen Produkten.

Die in den letzten Jahren permanent ansteigenden Anforderungen an hygienische Standards führen insbesondere auf dem Gebiet der Medizin zu einem erheblichen Bedarf an antimikrobiellen Materialien. Da gewöhnliche Verbrauchsmaterialien, beispielsweise Holz, Keramik, Kunststoff, Glas oder Stahl selbst keine antimikrobiellen Eigenschaften besitzen, müssen sie antimikrobiell gemacht werden.

Ein leistungsstarker Ansatz hierzu basiert auf sogenannten kontaktaktiven Systemen, wobei Materialien derart mit einer antimikrobiellen Modifikation versehen werden, daß Mikroorganismen bei Kontakt mit dem modifizierten Material getötet werden, ohne im Gegensatz zu den ebenso gebräuchlichen Freisetzungssystemen eine nur begrenzt vorhandene antimikrobielle Verbindung freizusetzen. Kontakt-aktive Systeme bestehen häufig aus aufgepropften antimikrobiellen Polymeren, insbesondere polykationischen Polymeren mit Ammonium-, Pyridinium-, Biguanidin-, Sulfonium- oder Phosphoniumgruppen. Allerdings erfordert die Aufbringung der Polymere auf das betreffende Material nicht selten aufwendige Oberflächenmodifizierungen. So ist beispielsweise aus der US 2004/0171978 A1 bekannt, daß für die Immobilisierung von Polylysin auf eine Polymeroberfläche zuerst eine Sulfonierung der Oberfläche durchgeführt wird. Außerdem sind einige kontakt-aktive Systeme aufgrund von toxischen Eigenschaften mancher antimikrobieller Polymere für medizinische Anwendungen nur eingeschränkt einsatzfähig.

Im medizinischen und klinischen Bereich bestehen jedoch hohe Anforderungen an die Bioverträglichkeit von Materialien, insbesondere von solchen, die für einen chirurgischen Einsatz bestimmt sind. Häufig verbleibt daher lediglich ein kleiner Spielraum zwischen antimikrobieller Wirksamkeit und Bioverträglichkeit des betreffenden Materials.

Aufgabe der vorliegenden Erfindung ist es daher, medizintechnische Produkte mit hoher antimikrobieller Wirksamkeit einerseits und hoher Bioverträglichkeit andererseits bereitzustellen.

In der WO 2004/056404 ist eine Kombination aus Metallnanopartikeln und Makromolekülen in Form eines polykationischen Biopolymers beschrieben, das unter anderem auch Poly-ε-Lysin umfassen kann. Poly-ε-Lysin hat zwar ein gutes Bindungsvermögen für Metalle in Nanopartikelform, wie zum Beispiel Silber. Die Kombination lässt sich jedoch kaum in organischen Lösungsmitteln, insbesondere unpolaren organischen Lösungsmitteln lösen, wodurch die Verarbeitung erschwert ist. Ähnliches ergibt sich aus der WO 2004/056403.

In einer älteren nicht vorveröffentlichten Patentanmeldung DE 10 2005 044 360.5 wird vorge,schlagen, Polyaminosäuren amphiphil zu modifizieren, um sie dadurch in organischen unpolaren Lösungsmitteln lösbar zu machen und insbesondere die Kombination aus Polyaminosäure und Metallnanopartikel. Damit wurden gute Ergebnisse erreicht. Es wird jedoch angestrebt, diese Ergebnisse noch zu verbessern, insbesondere was das Aufnahmevermögen an Metallnanopartikeln betrifft.

Diese Aufgabe wird gelöst durch ein medizintechnisches Produkt mit einer antimikrobiellen bzw. bioziden Ausstattung aus einem Komplexmaterial aus Metallnanopartikeln und Makromolekülen, wobei die Makromoleküle mindestens teilweise aus einer Polyaminosäure gebildet werden, die in besonderer Weise amphiphil modifiziert ist.

Gegenstand der Erfindung ist ein medizintechnisches Produkt mit einer antimikrobiellen Ausstattung aus einem Komplexmaterial aus Metallnanopartikeln und Makromolekülen, wobei die Makromoleküle mindestens teilweise aus amphiphil modifizierten Polyaminosäuren gebildet werden, die Aminosäurebausteine enthalten, die außer den in die Peptidbindung eingebundenen funktionellen Gruppen mindestens eine weitere funktionelle Gruppe aufweisen und mindestens ein Teil dieser Gruppen mit hydrophobe Reste tragenden Substanzen über kovalene Bindungen modifiziert sind.

Die Besonderheit bei der Erfindung gegenüber der älteren nicht vorveröffentlichten Anmeldung liegt darin, dass die hydrophoben Reste durch Reaktion von mindestens eine Epoxy-Gruppe tragenden Substanzen mit der mindestens einen weiteren funktionellen Gruppe und/oder mit mindestens einer Aminogruppe eines mindestens eine Aminogruppe als weitere funktionelle Gruppe tragenden Aminosäurebausteins unter Aufrechterhaltung einer Aminofunktion an die Polyaminosäuren gebunden sind.

Bei der älteren veröffentlichten Anmeldung DE 10 2005 044 360.5 erfolgt die Bindung der hydrophoben Reste an die zusätzlichen funktionellen Gruppen der Polyaminosäuren durch Ankoppeln von Säurechloriden insbesondere Fettsäurechloriden. Dadurch entstehen als Bindungsgruppen Ester, Thioester und insbesondere Amide. Der dadurch bedingte Verbrauch an funktionellen Gruppen bedingt eine Verminderung des Aufnahmevermögens an Metallnanopartikeln. Deshalb wird ein Bereich der Modifizierung gewählt, der eine zufriedenstellende Löslichkeit in unpolaren organischen Lösungsmitteln (z.B. Toluol) bringt und auf der anderen Seite ein ausreichendes Aufnahmevermögen für Metallpartikel gewährleistet.

Gemäß der Erfindung ist demgegenüber vorgesehen, die funktionelle Gruppe in ihrer Funktion möglichst wenig zu beeinträchtigen. Deshalb wird ein Wasserstoffatom oder ein anderer substituierbarer Rest an der funktionellen Gruppe lediglich durch eine den hydrophoben Rest tragende Substanz substituiert. Dadurch wird der Charakter der funktionellen Gruppe möglichst wenig beeinträchtigt.

Die den hydrophoben Rest tragende Substanz kann in der folgenden Weise mit einem mindestens eine weitere trifunktionelle Gruppe tragenden Aminosäurebaustein verbunden sein I) 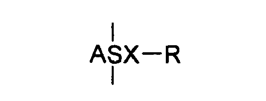 wobei AS der Aminosäurebaustein der Polyaminosäuren ist.

X ist -O-,-S-,-COO- und insbesondere -N(R₁)-, wobei R₁ ein aliphatischer Rest, ein Teil eines heterozyklischen Ringes und insbesondere H ist. R ist der hydrophobe Rest. Dieser kann ein aliphatischer Rest mit 8 bis 24 Kohlenstoffatomen sein. Vorzugsweise hat der Rest R folgende Formel 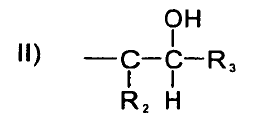
R₂ ist ein hydrophober, insbesondere aliphatischer, Rest mit 1 bis 10 Kohlenstoffatomen und insbesondere Wasserstoff.
R₃ ist ein hydrophober, insbesondere aliphatischer Rest mit 8 bis 24 Kohlenstoffatomen oder 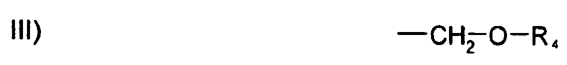
wobei R₄ ein hydrophober, insbesondere aliphatischer Rest mit 8 bis 24 Kohlenstoffatomen ist, der linear oder verzweigt sein kann.

Bei der Substituierung gemäß der Formel II handelt es sich um eine Substituierung an der weiteren funktionellen Gruppe durch eine eine Epoxy-Gruppe tragende hydrophobe Substanz, insbesondere eine solche mit einer endständigen Epoxy-Gruppe.
Bei einer Substitution gemäß einer Kombination der Formeln II und III handelt es sich um eine Epoxysubstitution unter Anwendung eines Glycidylethers. Eine solche Substitution zur Modifizierung ist bevorzugt, weil sich Glycidylether einerseits leicht herstellen lassen und andererseits ein biologischer Abbau über Glycerin und einen Alkohol möglich ist. Bei der Epoxy-Verbindung kann es sich auch um ein Diepoxid, insbesondere um einen Diglycidyldiether handeln.

Unter einer antimikrobiellen bzw. bioziden Ausstattung im Sinne der vorliegenden Erfindung soll eine Ausstattung verstanden werden, welche Zellwachstum und/oder -proliferation von Mikroorganismen, insbesondere von Keimen (schädliche Mikroorganismen), verhindert und/oder die Abtötung von vorhandenen Mikroorganismenkolonien, insbesondere Keimkolonien oder Biofilmen, bewirkt.

Gemäß einer Ausführungsform des medizintechnischen Produktes ist die antimikrobielle Ausstattung in Form eines Komplexmaterials mit antimikrobiellen Eigenschaften zumindest auf einem Teil der Oberfläche des Produktes, insbesondere in Form einer Beschichtung, vorgesehen. Die antimikrobielle Ausstattung ist vorzugsweise zumindest auf einem Teil der Oberfläche des Produktes, insbesondere in Form einer Beschichtung, vorhanden. Vorzugsweise erstreckt sich die antimikrobielle Ausstattung über die gesamte Oberfläche des medizintechnischen Produktes. Das derart ausgestattete medizintechnische Produkt zeichnet sich vorteilhafterweise dadurch aus, daß eine hinreichend stabile Haftverbindung zwischen der Ausstattung und der Oberfläche des Produktmaterials besteht, so daß beispielsweise ein Ablösen, insbesondere Abwischen oder Abwaschen, der Ausstattung vom beschichteten Produkt verhindert und somit eine mittelfristige, langfristige und wirkungsvolle Prävention des medizintechnischen Produktes gegen mikrobielle Besiedelung, insbesondere nach erfolgter Applikation, gewährleistet wird. Die Haftverbindung kann auf elektrostatischen Anziehungskräften, Wasserstoffbrückenbindungen und/oder lipophilen Interaktionen, insbesondere Van-der-Waals-Kräften, beruhen.

Zusätzlich oder alternativ zu der soeben beschriebenen Ausführungsform kann es erfindungsgemäß vorgesehen sein, daß sich das Komplexmaterial innerhalb des Produktes befindet. Dies kann besonders vorteilhaft sein, wenn es sich bei dem Werkstoff des medizintechnischen Produktes um ein Polymer oder auch ein anderes Material handelt, dessen Herstellungsprozess die Einführung des Komplexmaterials in das Innere des Produktes erlaubt. Auf diese Weise kann eine gleichmäßig verteilte antimikrobielle Wirksamkeit des medizintechnischen Produktes erzielt werden.

In einer weiteren Ausführungsform ist jeder Metallnanopartikel von mindestens einer Polyaminosäure umgeben, wobei jeder Metallnanopartikel vorzugsweise von allen Seiten hüllenartig von mindestens einer Polyaminosäure umgeben ist. Bevorzugt ist der polare, insbesondere geladene, Teil der Polyaminosäure zu den Metallnanopartikeln hin orientiert und ermöglicht durch die im polaren Teil befindlichen Heteroatome bzw. Heteroatomgruppierungen, beispielsweise Stickstoff- und/oder Sauerstoffatome, koordinative bzw. donative Bindungen zu den Metallnanopartikeln. Erfindungsgemäß können die Metallnanopartikel auf diese Weise partiell positiv geladen sein. Die modifizierte Polyaminosäure stellt eine Matrix für die Metallpartikel auf dem medizintechnischen Produkt dar.

Die Polyaminosäuren können insbesondere Homo- oder HeteroPolyaminosäuren sein, wobei Homopolyaminosäuren besonders bevorzugt sind. Bei Heteropolyaminosäuren sind solche bevorzugt, die einen hohen Anteil an Aminogruppen als weitere funktionelle Gruppe tragen.
Auch bei den Homopolyaminosäuren sind solche mit Aminogruppen bevorzugt, da die Aminogruppen die Bindung der Metallpartikel begünstigt. Die Polyaminosäuren können aus natürlich vorkommenden und/oder synthetischen Aminosäuren bestehen, wobei natürlich vorkommende Aminosäuren, insbesondere α-Aminocarbonsäuren, insbesondere eine L-Konfiguration aufweisende α-Aminocarbonsäuren, bevorzugt sind. Bevorzugt handelt es ich bei mindestens einem Teil der Aminosäuren um mindestens trifunktionelle Aminosäuren. Unter einer trifunktionellen Aminosäure im Sinne der vorliegenden Erfindung soll eine Aminosäure verstanden werden, welche zusätzlich zu der Carboxyl- und α-Aminogruppe eine weitere organische funktionelle Gruppe, insbesondere eine weitere Carboxyl-, Hydroxyl-, Thiol-, Guanidin- oder Aminogruppe, vorzugsweise eine weitere Aminogruppe, aufweist. Vorzugsweise enthalten die Polyaminosäuren mindestens eine basische, saure und/oder schwefelhaltige Gruppe tragende Aminosäure. Bei der schwefelhaltigen Gruppe kann es sich insbesondere um Thiol- und/oder Thioethergruppen handeln. Besonders bevorzugt enthalten die Polyaminosäuren mindestens eine Aminosäure aus der Gruppe, umfassend Cystein, Methionin, Tryptophan, Histidin, Arginin, Lysin, Ornithin, Asparaginsäure, Glutaminsäure und deren Derivate. Vorzugsweise sind mindestens 50 % der Aminosäurebausteine der Polyaminosäuren solche Aminosäuren, wobei Ornithin und insbesondere Lysin bevorzugt sind. Die restlichen Aminosäuren können Aminosäuren ohne zusätzliche funktionelle Gruppen, insbesondere Alanin, Valin, Leucin, Isoleucin und/oder Phenylalanin sein.

Die Polyaminosäure kann eine lineare Struktur aufweisen. Die lineare Struktur erlaubt eine dichte Anordnung um die zu stabilisierenden Metallnanopartikel, wobei die Anordnung insbesondere durch elektrostatische Kräfte, Wasserstoffbrückenbindungen und/oder lipophile Wechselwirkungen, insbesondere Van-der-Waals-Kräfte, stabilisiert wird.

In einer anderen bevorzugten Ausführungsform der Erfindung weisen die Polyaminosäuren eine verzweigte, vorzugsweise eine hyperverzweigte, Struktur auf. Bei der Herstellung der Polyaminosäuren kann auch eine interne Vernetzung stattfinden. Die verzweigte und/oder vernetzte Struktur erlaubt insbesondere eine kompakte Anordnung um die zu stabilisierenden Metallnanopartikel. Die verzweigten Polyaminosäuren besitzen mindestens teilweise globuläre Struktur, durch die insbesondere die Stabilisierung der Metallnanopartikel erhöht wird. Weiterhin bewirkt die verzweigte Struktur der Polyaminosäuren in vorteilhafter Weise eine verringerte Sprödigkeit des Komplexmaterials des erfindungsgemäßen Produktes. Somit erhöhen verzweigte und/oder vernetzte Polyaminosäuren in bevorzugter Weise die filmbildenden Eigenschaften des Komplexmaterials. Es können auch verschiedene Polyaminosäuren in Mischung vorliegen.

Bei einer bevorzugten Ausführungsform der Erfindung bestehen die Makromoleküle aus den Polyaminosäuren. Weiterhin sind die Polyaminosäuren mit besonderem Vorteil ausschließlich aus Aminosäuren mit mindestens einer weiteren funktionellen Gruppe gebildet. Wiederum bevorzugt sind Polyaminosäuren, die im noch nicht modifizierten Zustand ausschließlich aus Aminosäurebausteinen bestehen, bei denen die mindestens eine weitere funktionelle Gruppe eine nukleophile Gruppe insbesondere eine Aminogruppe ist.

Als weitere funktionelle Gruppen sind primäre Aminogruppen besonders bevorzugt. Diese werden bei der erfindungsgemäßen Substitution in sekundäre Aminogruppen umgewandelt. Solche sekundären Aminogruppen tragen mit zur Metallbindung bei, da das sehr gute Bindungsvermögen von primären Aminogruppen durch die Umwandlung in sekundäre Aminogruppen kaum beeinträchtigt wird. In entsprechender Weise sind bei einer besonders bevorzugten Ausführungsform der Erfindung mindestens 50 % der weiteren funktionellen Gruppen der Polyaminosäuren primäre Aminogruppen.

Gemäß einer bevorzugten Ausführungsform des medizintechnischen Produktes ist die Polyaminosäure Polylysin, insbesondere Poly-α-Lysin (P-oly-alpha-Lysin) und/oder Poly-s-Lysin (Poly-epsilon-Lysin). Sowohl Poly-α-Lysin als auch Poly-ε-Lysin weisen antimikrobielle Eigenschaften auf, wobei Poly-ε-Lysin im Gegensatz zu Poly-α-Lysin bioverträglicher und daher besonders bevorzugt ist.

Die nicht modifizierte Polyaminosäure, insbesondere das Polylysin, des erfindungsgemäßen Produktes hat vorzugsweise ein Molekulargewicht im Bereich von 500 bis 1 000 000 g/mol, insbesondere 3000 bis 100 000 g/mol. Der jeweils gewünschte Molekulargewichtsbereich kann durch die Art der Herstellung der Polyaminosäure eingestellt werden.

Beim erfindungsgemäßen medizintechnischen Produkt ist die Polyaminosäure mit einer Substanz mit mindestens einem hydrophoben Rest, insbesondere mit einer solchen mit mindestens einem aliphatischen Rest, amphiphil modifiziert. Eine derartige Modifizierung dient einerseits dazu, in der Lösung eine Stabilisierung der Metallnanopartikel und die gegenseitige Abschirmung der die Nanopartikel koordinierenden Polyaminosäuren zu erhöhen und andererseits dazu, eine gute Löslichkeit iin organischen, insbesondere unpolaren Lösungsmitteln zu erreichen. Auf diese Weise kann die Entstehung größerer Nanopartikel, insbesondere in Form von Aggregaten, verhindert werden. Weiterhin kann die Bildung von Aggregaten von Polyaminosäuren vermieden werden. Vorzugsweise ist der hydrophobe Rest nach Modifizierung der Polyaminosäure insbesondere von den Metallnanopartikeln weg nach außen orientiert. Die so erhaltene Struktur aus Metallnanopartikeln und amphiphil modifizierten Polyaminosäuren kann als sogenannte Core-Shell-Struktur (Kern-Hülle-Partikel) bezeichnet werden, wobei die die Metallnanopartikel unmittelbar umgebenden Polyaminosäuren den Kern und die hydrophobe Substanz die Schale der Struktur darstellen. Unter Amphiphilität im Sinne der vorliegenden Erfindung soll die Eigenschaft einer Verbindung verstanden werden, welche aufgrund ihrer molekularen Struktur sowohl hydrophile als auch lipophile Eigenschaften aufweist. Ein Komplex mit einer Core-Shell-Struktur ist insbesondere aus der DE 103 23 597 A1 bekannt, die im Wesentlichen aus amphiphil modifiziertem Polyethylenimin besteht.

Ein großer Vorteil der Erfindung liegt darin, dass der Substitutionsgrad der Polyaminosäuren im Rahmen der Modifizierung mit den hydrophoben Substanzen in weiten Grenzen gehalten werden kann und dabei, wie bereits erwähnt, ein hohes Aufnahmevermögen an Metallnanopartikeln erzielt wird. Normalerweise liegt der Substitutionsgrad bei der Modifizierung im Rahmen von 10 bis 80 % bezogen auf die in der nicht modifizierten Polyaminosäure vorhandenen weiteren funktionellen Gruppe.

Bevorzugt weist der aliphatische Rest der Substanz 8 bis 24, insbesondere 12 bis 20, vorzugsweise 16 und/oder 18, Kohlenstoffatome auf. Bei dem aliphatischen Rest kann es sich um ein Alkyl-, Alkenyl und/oder einen Alkinylsubstituenten handeln, wobei Alkylsubstituenten, insbesondere unverzeigt, besonders bevorzugt sind. So erlauben Alkylsubstituenten, insbesondere langkettige und vorzugsweise unverzweigte Alkylsubstituenten, im Bereich der Schale der Core-Shell-Struktur eine dichtere bzw. kompaktere Anlagerung der Alkylketten aneinander und eine attraktive Wechselwirkung mit dem medizinischen Produkt. In organischer Lösung stoßen die Alkylsubstituenten die Partikel voneinander ab und verhindern so eine Agglomeration.

Gemäß der Erfindung handelt es sich bei der Substanz zur amphiphilen Modifikation um mindestens eine bioverträgliche Substanz. Für die amphiphile Modifikation der Polyaminosäure liegt die Substanz in einer aktiven Form, insbesondere als Epoxid vor. Weiterhin kann es erfindungsgemäß bevorzugt sein, daß die Substanz als Gemisch verschiedener Substanzen vorliegt.

Glycidylether, insbesondere solche mit aliphatischen Resten, sind als Substanzen für die Modifizierung besonders geeignet. Einerseits lassen sie sich in einfacher Weise herstellen. Auf der anderen Seite lassen sie sich auch in einfacher Weise mit der Polyaminosäure umsetzen. Auch ihr Abbau ist, wie bereits erwähnt, unproblematisch.

Besonders geeignete Substanzen für die Modifizierung sind Glycidylhexadecylether und Glycidyloctadecylether.

Es ist in besonderen Fällen auch möglich zusätzliche zur Modifikation mit Epoxiden eine solche mit Fettsäurechloriden vorzunehmen.

Gemäß einer besonders bevorzugten Ausführungsform des erfindungsgemäßen Produktes beruht die amphiphile Modifikation der Polyaminosäure mit der Substanz auf kovalenten Bindungen, insbesondere auf Aminobindungen. Vorzugsweise sind sekundäre Amine aus den freien Aminogruppen der Polyaminosäure und Epoxygruppen der Substanz gebildet. Im Falle des Poly-ε-Lysins handelt es sich bei den freien Aminogruppen um die α-Aminogruppen der Polyaminosäure. Das medizintechnische Produkt zeichnet sich vorzugsweise dadurch aus, daß der Anteil an freien Aminogruppen der Polyaminosäure nach der amphiphilen Modifikation der Polyaminosäure zwischen 0,5 und weniger als 50%, insbesondere zwischen 10 und 40%, insbesondere zwischen 20 und 40%, vorzugsweise bei ca. 27%, liegt, bezogen auf die ursprünglichen freien Aminogruppen der Polyaminosäure insbesondere des Poly-Lysins.

Gemäß einer weiteren besonders bevorzugten Ausführungsform des medizintechnischen Produktes sind die Polyaminosäuren vernetzt, insbesondere über eine polyfunktionelle Carbonsäure, vorzugsweise Zitronensäure. Die Vernetzung beruht vorzugsweise auf der Bildung von kovalenten Bindungen, insbesondere Amidbindungen, wobei die Amidbindungen durch Kondensation zwischen Aminogruppen der Polyaminosäure und Säuregruppen, insbesondere Carboxylgruppen, der vernetzenden Komponente, gebildet sind. Die Vernetzung der Polyaminosäure ist besonders vorteilhaft, da auf diese Weise nach der amphiphilen Modifikation der vernetzten Polyaminosäure spherische Strukturen insbesondere geschlossene Core-Shell-Strukturen vorliegen und die funktionellen Gruppen, insbesondere Carboxylgruppen, der vernetzenden Komponente die Anzahl an möglichen Koordinationsstellen für die Metallnanopartikel im Polymer erhöhen. Eine Vernetzung ist insbesondere bei linearen Polyaminosäuren von Vorteil. Auf diese Weise können die Komplexierungseigenschaften für die Metallnanopartikel verbessert werden. Weiterhin können durch die Vernetzung bestimmte Eigenschaften des bioziden Komplexmaterials, insbesondere dessen filmbildenden Eigenschaften, verbessert werden. Bei verzweigten Polyaminosäuren liegen spherische Strukturen auch ohne Vernetzung vor.

Bevorzugt liegt der Anteil an freien Aminogruppen der Polyaminosäure nach Vernetzung der Polyaminosäure, insbesondere mit Zitronensäure, zwischen 25 und weniger als 50%, insbesondere zwischen 30 und 45%, vorzugsweise zwischen 35 und 43%, bezogen auf die ursprüngliche Aminogruppen-Gesamtmenge der zur Herstellung der Polyaminosäure verwendeten Aminosäure-Monomere, vorzugsweise Lysin-Monomere. Vorzugsweise weist ein mit 5 mol-% Zitronensäure bezüglich der eingesetzten Lysin-Monomere vernetztes Polylysin, insbesondere Poly-ε-Lysin, einen Anteil an freien Aminogruppen von ca. 43% und ein mit 10 mol-% Zitronensäure bezüglich der eingesetzten Lysin-Monomere vernetztes Polylysin, insbesondere Poly-ε-Lysin, einen Anteil an freien Aminogruppen von ca. 35% auf, bezogen auf die ursprüngliche Aminogruppen-Gesamtmenge der zur Herstellung der Polyaminosäure verwendeten Lysin-Monomere.

In einer weiteren bevorzugten Ausführungsform liegt der Anteil an freien Aminogruppen der Polyaminosäure nach der amphiphilen Modifikation der vernetzten Polyaminosäure zwischen 15 und 35%, insbesondere zwischen 25 und 35%, vorzugsweise bei ca. 30%, bezogen auf die ursprüngliche Aminogruppen-Gesamtmenge der zur Herstellung der Polyaminosäure verwendeten Aminosäuren-Monomere, vorzugsweise Lysin-Monomere. In einer besonders bevorzugten Ausführungsform weist ein mit 5 mol-% Zitronensäure bezüglich der eingesetzten Lysin-Monomere vernetztes Polylysin, insbesondere Poly-ε-Lysin, nach der amphiphilen Modifikation einen Anteil an freien Aminogruppen von ca. 32% und ein mit 10 mol-% Zitronensäure bezüglich der eingesetzten Lysin-Monomere vernetztes Polylysin, insbesondere Poly-ε-Lysin, nach der amphiphilen Modifikation einen Anteil an freien Aminogruppen von ca. 26% auf, bezogen auf die ursprüngliche Aminogruppen-Gesamtmenge der zur Herstellung der Polyaminosäure verwendeten Lysin-Monomere. Die Vernetzung kann in vorteilhafterweise auch mit mehrfunktionellen Epoxyden vorgenommen werden, insbesondere mit aliphatischen Diepoxyden, wobei die Epoxygruppe endständig angeordnet ist, was insgesamt bei den Epoxyden bevorzugt ist. Die Vernetzung mit Epoxyden hat wiederum den Vorteil, dass die funktionellen, Gruppen durch die Epoxydanlagerung in ihrer Funktion wenig beeinträchtigt werden. Außerdem kann der Epoxydvernetzer durch Vorhandensein eines hydrophoben Restes zwischen den beiden Epoxygruppen auch zur Hydrophobisierung der Polyaminosäure, d.h. zur weiteren Modifizierung beitragen. Es ist auch denkbar, die Modifizierung ausschließlich über eine hydrophobierende Vernetzung vorzunehmen, z.B. mit hydrophoben aliphatischen Diglycidyldiethern mit 8 bis 24 Kohlenstoffatomen. Epoxide, insbesondere Diepoxide, mit einer mittelständigen cis-Doppelbindung können die Löslichkeit in organischen Lösungsmitteln durch ihre Konfiguration besonders unterstützen.

Im Falle der Metallnanopartikel kann es sich erfindungsgemäß um Gold-, Silber-, Kupfer- oder Zinknanopartikel handeln, wobei Silbernanopartikel besonders bevorzugt sind. Mit Vorteil weisen die Metallnanopartikel einen Durchmesser von 0,5 bis 20 nm, insbesondere 1 bis 20 nm, vorzugsweise 1 bis 14 nm, auf.

Vorzugsweise weisen Nanosilberpartikel, die durch amphiphil modifizierte Polyaminosäuren, insbesondere Polylysin, vorzugsweise Poly-ε-Lysin, stabilisiert sind, einen Durchmesser von ca. 6 nm auf, insbesondere nach Reduktion mit Ascorbinsäure. In manchen Fällen kann es jedoch bevorzugt sein, daß die Nanosilberpartikel einen kleineren Durchmesser, insbesondere von ca. 4 nm, aufweisen. Dies ist beispielsweise durch eine Reduktion mit dem Reduktionsmittel LiBHEt₃ möglich.

Gemäß einer anderen bevorzugten Ausführungsform weisen Nanosilberpartikel, die durch vernetzte, insbesondere durch Zitronensäure vernetzte, amphiphil modifizierte Polyaminosäuren, insbesondere Polylysin, vorzugsweise Poly-ε-Lysin, stabilisiert sind, einen Durchmesser von ca. 10 nm (5 mol-% Zitronensäure bezüglich der eingesetzten Lysin-Monomere) oder ca. 8 nm (10 mol-% Zitronensäure bezüglich der eingesetzten Lysin-Monomere) auf. Dies kann beispielsweise durch Reduktion mit Ascorbinsäure erreicht werden.

In manchen Fällen kann es wünschenswert sein, dass die Metallnanopartikel, insbesondere Silbernanopartikel, des Komplexmaterials einen Durchmesser im Bereich von 2,5 bis 3,5 nm aufweisen. Dies kann beispielsweise durch Verwendung von Polylysin, vorzugsweise von Poly-ε-Lysin, erreicht werden. Bevorzugt ist das Polylysin mit Zitronensäure vernetzt und modifiziert. So können die Metallnanopartikel im Falle einer Stabilisierung durch Polylysin, welches mit 5 mol-% Zitronensäure bezüglich der eingesetzten Lysin-Monomere vernetzt ist, einen Durchmesser von ca. 3,1 nm aufweisen. Im Falle der Verwendung von Polylysin, welches mit 10 mol-% Zitronensäure bezüglich der eingesetzten Lysin-Monomere vernetzt ist, können die Metallnanopartikel einen Durchmesser von ca. 2,7 nm aufweisen.

Erfindungsgemäß kann es weiterhin vorgesehen sein, daß es sich bei dem medizintechnischen Produkt um ein temporäres oder dauerhaftes lmplantat für den menschlichen oder tierischen Körper handelt. Hierbei handelt es sich bei den antimikrobiell ausgestatteten Implantaten vorzugsweise um Katheter, Gelenkimplantate, Stents, Schrauben, Nägel und Platten. Die Implantate können aus Metallen oder Metalllegierungen bestehen. Als weitere Materialien für die Implantate kommen insbesondere Kunststoffe in Betracht. Die Implantate können beispielsweise zur Reparatur von Frakturen verwendet werden. Bei den Implantaten kann es sich weiterhin um Netze, vorzugsweise um Herniennetze, handeln. Als weitere bevorzugte Implante kommen insbesondere Gefäßprothesen, Patchs, Nahtmaterialien, Membranen sowie Folien, beispielsweise zur Adhäsionsprophylaxe, in Betracht. Erfindungsgemäss ist es weiterhin bevorzugt, dass es sich bei den Implantaten um Inkontinenzbänder sowie allgemein um textile Implantate handeln kann. Als textile Implantate kommen insbesondere Gewebe, Gestricke, Gewirke, Geflechte, Gelege und Vliese in Frage. Die biozide Ausstattung dieser Implantate ermöglicht es, diese auch in akut infizierte oder infektions-gefährdete Körperregionen einzuführen, da die Implantate selbst durch die Ausstattung antimikrobiell wirken und auf diese Weise zu einer Verringerung einer vorhandenen oder potentiellen Infektion beitragen.

In einer anderen Ausführungsform handelt es sich bei den medizintechnischen Produkten um medizinische Instrumente, insbesondere um chirurgische Scheren, Zangen und Klammern sowie um Katheter oder Sonden und weitere Instrumente, insbesondere für minimalinvasive Eingriffe. In diesem Zusammenhang ist die bereits erwähnte Haftverbindung der antimikrobiellen Ausstattung mit der Oberfläche des medizintechnischen Produktes von besonderem Vorteil, da die beispielsweise soeben bezeichneten medizinischen Instrumente einer besonders hohen mechanischen Beanspruchung, insbesondere durch Reiben und Wischen, ausgesetzt sind. Die Haftung der antimikrobiellen Ausstattung mit der Produktoberfläche wird insbesondere durch lipophile Wechselwirkungen, vorzugsweise Van-der-Waals-Kräfte, der insbesondere von den Metallnanopartikeln wegweisenden hydrophoben Reste, insbesondere langkettigen aliphatischen Reste der Substanz mit der Produktoberfläche bewirkt.

Bei den medizintechnischen Produkten gemäß der vorliegenden Erfindung kann es sich ferner um Erzeugnisse wie beispielsweise Drainageschläuche, Nahtmaterialien oder Wundauflagen handeln. Bei dem Material des medizintechnischen Produktes handelt es sich gemäß einer weiteren bevorzugten Ausführungsform um ein Metall oder eine Metalllegierung, insbesondere um Titan, Edelstahl, Magnesium, Tantal oder einer Legierung davon, wobei Magnesium und/oder Tantal wegen ihrer Bioverträglichkeit und Resorbierbarkeit besonders bevorzugt sind.

In einer weiteren Ausführungsform handelt es sich bei dem Material des medizintechnischen Produktes um ein nichtresorbierbares oder um ein mindestens teilweise resorbierbares Polymer. So kann es sich bei dem nichtresorbierbaren Polymer um ein Polyolefin, insbesondere Polyethylen und/oder Polypropylen, einen Polyester, insbesondere Polyethylenterephthalat und/oder Polybutylenterephthalat, ein Polyamid, insbesondere Polyamid-6 oder Polyamid-6,6, oder eine Naturfaser, insbesondere Seide oder Leinen, handeln. Bevorzugt ist das mindestens teilweise resorbierbare Polymer ein vollständig resorbierbares Polymer. Bei dem resorbierbaren Polymer kann es sich insbesondere um ein Polymer auf der Basis der Monomere Lactid, Glykolid, Trimethylencarbonat, para-Dioxanon, ε-Caprolacton und/oder Hydroxybutyrat, vorzugsweise in Form eines Co- und/oder Terpolymers, handeln. Entsprechend einer weiteren Ausführungsform kann ein medizintechnisches Produkt, dessen Material nicht resorbierbar ist, mit einem mindestens teilweise resorbierbaren, vorzugsweise vollständig resorbierbaren, Polymer, insbesondere mit einem der soeben aufgeführten Polymere, beschichtet werden, um somit die Zutrittsdauer von Flüssigkeiten, insbesondere von Körperflüssigkeiten, zu der antimikrobiellen Ausstattung zu beeinflußen bzw. zu regulieren.

In einer weiteren Ausführungsform handelt es sich bei dem Material des medizintechnischen Produktes um einen keramischen Werkstoff. Mit Vorteil kann es sich um einen resorbierbaren keramischen Werkstoff, insbesondere um Hydroxylapatit oder Tricalciumphosphat, handeln.

Gemäß einer weiteren Ausführungsform weist das Produkt Poren, vorzugsweise interkonnektierende Poren, auf. Dies kann besonders vorteilhaft sein, da auf diese Weise eine vergrößerte Oberfläche für die antimikrobielle Ausstattung zur Verfügung steht. Somit kann eine größere Menge des bioziden bzw. antimikrobiellen Komplexmaterials auf und, im Falle eines interkonnektierenden Porensystems, auch innerhalb des auszustattenden Produktes aufgebracht werden.

Das Produkt ist mit Vorteil sterilisierbar und liegt insbesondere in sterilisierter Form vor. Als Sterilisierungsmethoden kommen alle dem Fachmann bekannten Methoden, insbesondere Bestrahlung, Hitzesterilisation, Dampfsterilisation, Ethylenoxidbegasung und Plasmasterilisation in Frage, die vorzugsweise die chemische Struktur und/oder die antimikrobiellen Eigenschaften des insbesondere in Form einer Core-Shell-Struktur vorliegenden Komplexmaterials nicht beeinträchtigen. Das erfindungsgemäße medizintechnische Produkt liegt im Gebrauchszustand vorzugsweise in steriler Form vor.

Der Gegenstand der Erfindung betrifft außerdem ein Verfahren zur Herstellung eines medizintechnischen Produktes, wobei das Komplexmaterial, insbesondere in Form einer Lösung, von außen auf das nicht ausgestattete Produkt aufgebracht wird. In der Lösung liegen die Metallnanopartikel, insbesondere Silbernanopartikel, vorzugsweise gemäß einer der vorhergehenden Ausführungsformen stabilisiert vor. Die Herstellung einer derartigen Lösung erfolgt vorzugsweise ausgehend von amphiphil modifizierten und insbesondere vernetzten Polyaminosäuren. Die so hergestellten Core-Shell-Polymere werden in einem organischen Lösungsmittel aufgelöst und durch Zugabe eines Metallsatzes mit dem entsprechenden Metallion beladen. Die auf diese Weise hergestellte Lösung enthält durch amphiphil modifizierte und insbesondere vernetzte und/oder verzweigte Polyaminosäuren stabilisierte Metallionen und eignet sich insbesondere für die antimikrobielle Ausstattung von medizintechnischen Produkten, insbesondere zur Ausstattung der bereits genannten medizintechnischen Produkte. Bevorzugt werden die derartig stabilisierten Metallionen der Lösung jedoch in Gegenwart eines geeigneten Reduktionsmittels, insbesondere Vitamin C, Natriumborhydrid, LiHBEt₃ oder einem Aldehyd, zu elementaren Metallnanopartikeln reduziert. Im Falle der Verwendung von LiHBEt₃ (Li: Lithium, H: Wasserstoff, B: Bor und Et: Ethyl) als Reduktionsmittel können davon abgeleitete Lithium-Bor-Spezies aufgrund der Oxophilie dieser Lithium-Bor-Spezies zu einer Vernetzung und damit zu einer Aggregation der amphiphil modifizierten und insbesondere vernetzten Polyaminosäuren führen. Daher und insbesondere wegen seiner Bioverträglichkeit ist die Verwendung von Vitamin C als Reduktionsmittel besonders bevorzugt. Interessanterweise wurde beim erfindungsgemäßen Komplexmaterial, insbesondere beim durch Epoxide modifizierten Komplexmaterial, auch eine Autoreduktion der Metallionen beobachtet, so dass auf die Verwendung eines Reduktionsmittels verzichtet werden kann. Als organische Lösungsmittel können diverse Alkohole, insbesondere Isopropanol oder Propanol, oder aromatische Lösungsmittel, beispielsweise Toluol oder Xylol, sowie Mischungen davon verwendet werden.

Weiterhin kann es bevorzugt sein, das antimikrobielle Komplexmaterial als Feststoff, beispielsweise durch Sputtering, oder in Form einer Schmelze oder eines Aerosols auf das auszustattende Produkt aufzubringen.

Vorzugsweise wird das antimikrobielle Komplexmaterial in einem Tauchverfahren auf die Oberfläche des nicht ausgestatteten Produktes aufgebracht. Weiterhin kann das antimikrobielle Komplexmaterial durch Quellung in und/oder auf das nicht ausgestattete Produkt gebracht werden. Für die antimikrobielle Ausstattung von beispielsweise Nahtmaterialien, Netzen oder Bändern kann es bevorzugt sein, das biozide Komplexmaterial im Durchzugsverfahren von außen auf das nicht ausgestattete Produkt aufzubringen. Weiterhin kann das biozide Komplexmaterial durch dem Fachmann bekannte Ausgieß-, Ausstreich-, Präge- und Sprühtechniken, insbesondere Pressen, Walzen oder Rakeln, auf das nicht ausgestattete Produkt aufgebracht werden.

Gemäß einer weiteren Ausführungsform wird zusätzlich ein mindestens teilweise resorbierbares, vorzugsweise vollständig resorbierbares Material, vorzugsweise ein Polymer, insbesondere in Form einer Lösung, auf die Oberfläche des Produktes aufgebracht. So kann es bevorzugt sein, daß das erfindungsgemäße Produkt nach einer oberflächlichen Beschichtung mit dem antimikrobiellen Komplexmaterial in einem zweiten Beschichtungsverfahren mit einer zweiten Schicht eines resorbierbaren Polymers versehen wird. Vorzugsweise wird als zweite Schicht ein resorbierbares Co- und/oder Terpolymer, insbesondere auf der Basis von Lactid, Glykolid, Trimethylencarbonat, Hydroxybutyrat, para-Dioxanon und/oder ε-Caprofacton, aufgebracht. Als resorbierbare Materialien eignen sich auch Fettalkohole, Wachse und Fettsäuresalze, insbesondere Stearat. Als Lösungsmittel können Alkohole, aliphatische Ester, Ketone oder aromatische Lösungsmittel eingesetzt werden, wobei Ethylacetat besonders bevorzugt ist. Weiterhin ist es möglich, daß das resorbierbare Polymer nach einer Oberflächenbehandlung des medizintechnischen Produktes, insbesondere nach einer Plasmaaktivierung, auf das Produkt aufgebracht wird.

Alternativ dazu kann das mindestens teilweise resorbierbare, vorzugsweise vollständig resorbierbare, Polymer und das antimikrobielle Komplexmaterial gemeinsam in einem Beschichtungsprozess auf das auszustattende medizintechnische Produkt aufgebracht werden. Dies ist besonders vorteilhaft, da ein einziger Beschichtungsprozess wirtschaftlicher und damit kostengünstiger ist.

Weiterhin ist es möglich, daß eine Keramik- und/oder Metallbeschichtung, insbesondere gemäß einer der beiden zuletzt beschriebenen Ausführungsformen, auf das auszustattende Produkt z.B. durch Plasmabedampfung aufgebracht wird.

Gegenstand der Erfindung ist weiterhin ein Verfahren zur Herstellung eines medizintechnischen Produktes, wobei das Komplexmaterial, insbesondere in Form einer Lösung, zusätzlich oder alternativ zu den vorherigen Ausführungsformen in den Werkstoff des Produktes bei dessen Herstellung zugegeben oder nach Herstellung des Produktes durch Quellung in dieses eingelagert wird. Durch die Zugabe zum Werkstoff des Produktes kann eine gleichmäßige Verteilung des antimikrobiellen Komplexmaterials innerhalb des medizintechnischen Produktes sowie auf dessen Oberfläche oder zumindest in Schichten nahe der Produktoberfläche erreicht werden. Bezüglich weiterer Einzelheiten, insbesondere in Bezug auf die Lösung sowie auf alternative Zugabeformen des antimikrobiellen Komplexmaterials zum Werkstoff des medizintechnischen Produktes, insbesondere als Feststoff oder in Form einer Schmelze oder eines Aerosols, wird auf die obige Beschreibung verwiesen.

In einer weiteren vorteilhaften Ausführungsform wird das antimikrobielle Komplexmaterial mit dem Produktwerkstoff gemischt und anschließend zum gewünschten Produkt geformt, insbesondere extrudiert, gesponnen, gepreßt, gewalzt, gegossen oder geblasen. Besonders bevorzugt wird eine Mischung aus Polymer und antimikrobiellem Komplexmaterial zu einem Fadenmaterial versponnen, welches je nach Art des verwendeten Polymers zu resorbierbarem oder zu nicht resorbierbarem Nahtmaterial oder zu einem textilen Produkt verarbeitet, insbesondere verwebt, verwirkt oder geflochten werden kann.

Die vorliegende Erfindung betrifft weiterhin ein Verfahren zur Herstellung von mindestens einer Polyaminosäure, insbesondere zur Herstellung des erfindungsgemäßen Produktes, durch Polymerisation von Aminosäuren mit mindestens einem Anteil an mindestens trifunktionellen Aminosäuren in flüssiger Phase, wobei die Aminosäuren ohne Verwendung von Schutzgruppen polymerisiert werden. Die Polymerisation, die im engeren Sinne eine Polykondensation ist, kann nach vorheriger Aktivierung der Aminosäuren oder ohne Aktivierung bei erhöhter Temperatur durchgeführt werden. Nachfolgend werden verschiedene Polymerisationsverfahren beschrieben. Diese können zu verschiedenen Ausbeuten und zu verschiedenen Polyaminosäuren (linear, verzweigt, sterisch rein, racemisiert, niedermolekular, hochmolekular) führen.

In einer Ausführungsform des erfindungsgemäßen Verfahrens werden zur Bereitstellung der flüssigen Phase organische Lösungsmittel oder organische Lösungsmittelgemische verwendet. Mit Vorteil wird die Polymerisation der Aminosäuren in mindestens einem Lösungsmittel, ausgewählt aus der Gruppe, umfassend Dimethylsulfoxid (DMSO), Dimethylformamid (DMF), Dichlormethan, Tetrahydrofuran (THF), Toluol, Xylol und Ethylacetat, vorgenommen.

In einer bevorzugten Ausführungsform des erfindungsgemäßen Verfahrens werden die Aminosäuren durch mindestens einen Stoff, vorzugsweise einen organischen Stoff, aktiviert. Mit besonderem Vorteil wird als Stoff eine stickstoffhaltige Verbindung verwendet. Bevorzugt werden die Aminosäuren durch Reaktion mit dem Stoff in besonders reaktive Zwischenprodukte (Intermediate), insbesondere in Aktivester, überführt und somit aktiviert. Die auf diese Weise aktivierten Aminosäuren können mit nukleophilen Gruppen, insbesondere mit Aminogruppen, von anderen Aminosäuren reagieren. Dadurch kann die Polymerisation der Aminosäuren zu der Polyaminosäure unter besonders milden Reaktionsbedingungen, insbesondere bei Raumtemperatur, vorgenommen werden. Unter solchen milden Bedingungen bleibt die Stereochemie der Aminosäuren erhalten.

In einer besonderen Ausführungsform des erfindungsgemäßen Verfahrens werden die Aminosäuren durch mindestens einen Stoff aus der Gruppe, umfassend Carbodiimide, N-Hydroxysuccinimid (NHS), 1-Hydroxybenzotriazol (HOBT) und davon abgeleitete Derivate, aktiviert. Weiterhin können die Aminosäuren durch Reaktion mit Pentachlorophenol und/oder Pentafluorophenol aktiviert werden. Als Carbodiimide können insbesondere 1-Ethyl-3-(3-dimethylaminopropyl)-carbodiimid (EDC) oder Dicyclohexylcarbodiimid (DCC) verwendet werden. Die Verwendung von EDC kann insbesondere wegen seiner guten Wasserlöslichkeit bevorzugt sein. So kann beispielsweise das nach erfolgreicher Polymerisation entstandene und von EDC abgeleitete lsoharnstoffderivat durch einfache Reinigungsschritte, insbesondere durch eine wäßrige Extraktion, aus dem Reaktionsansatz entfernt werden. In einer anderen Ausführungsform kann es sinnvoll sein, die Aminosäuren für die Polymerisation durch DCC zu aktivieren.

In einigen Fällen ist das Aktivierungspotenzial von Carbodiimiden, insbesondere von DCC, für eine zufriedenstellende Polymerisation nicht ausreichend, da beispielsweise die durch Carbodiimide aktivierte Zwischenprodukte insbesondere durch Folgereaktionen deaktiviert werden können. Bei diesen unerwünschten Folgereaktionen kann es sich insbesondere um Umlagerungsreaktionen handeln, wodurch die aktivierten Intermediate, insbesondere Aktivester, in deaktivierte Produkte, insbesondere Amidverbindungen, überführt werden, ehe sie zur gewünschten Polyaminosäure polymerisieren können. In solchen Fällen kann es erfindungsgemäß sinnvoll sein, die Aminosäuren durch Carbodiimide, insbesondere DCC, und einem weiteren Stoff zu aktivieren. Vorzugsweise reagiert der weitere Stoff mit den aus den Carbodiimiden und den Aminosäuren gebildeten aktivierten Zwischenprodukten unter Bildung von neuen, vorzugsweise reaktiveren, Zwischenprodukten, insbesondere unter Bildung von Aktivestern. Die auf diese Weise hergestellten aktiveren Zwischenprodukte reagieren besonders schnell mit den Aminogruppen der für die Polymerisation eingesetzten Aminosäuren. Auf diese Weise kann mit besonderem Vorteil eine Deaktivierung von aktiven Zwischenprodukten vermieden werden. Insbesondere ist somit die Herstellung einer Polyaminosäure, beispielsweise einer Polyaminosäure mit einem gewünschten DP (Degree Of Polymerisation), möglich.

In einer besonders bevorzugten Ausführungsform des erfindungsgemäßen Verfahrens werden die Aminosäuren durch Dicyclohexylcarbodiimid (DCC) und N-Hydroxysuccinimid (NHS) aktiviert.

In einer anderen bevorzugten Ausführungsform des erfindungsgemäßen Verfahrens werden die Aminosäuren durch Dicyclohexylcarbodümid (DCC) und 1-Hydroxybenzotriazol (HOBT) aktiviert.

Weiterhin können die Aminosäuren durch Thionylchlorid (SOCl₂) in DMF als Lösungsmittel aktiviert werden, wobei die Aktivierung vorzugsweise über eine DMF-katalysierte Umsetzung mit Thionylchlorid (SOCl₂) vorgenommen wird.

Weiterhin können die Aminosäuren durch Erhitzen in flüssiger Phase (z.B. Wasser) polymerisiert werden. Mit besonderem Vorteil wird durch das Erhitzen eine Kondensationsreaktion zwischen den Carboxyl- und Aminogruppen der Aminosäuren unter Abspaltung von Wasser und Ausbildung von Amid-Bindungen vorgenommen. Die Kondensationsreaktion wird insbesondere bei einer Temperatur (Reaktionstemperatur) zwischen 100 und 170 °C, insbesondere zwischen 110 und 160 °C, durchgeführt. Die Verknüpfung der Aminosäure-Monomere wird insbesondere während eines Zeitraums von 8 bis 96 Stunden, vorzugsweise während ca. 96 Stunden, vorgenommen. Mit Vorteil kann die Reaktionsführung bei unterschiedlichen Temperaturen, insbesondere bei zwei unterschiedlichen Temperaturen, durchgeführt werden. Mit besonderem Vorteil kann die Polymerisation bei Temperaturen von ca. 160 °C und ca. 120 °C durchgeführt werden. Bevorzugt wird der Reaktionsansatz zunächst auf eine Temperatur von ca. 140 °C gebracht und während ca. 48 Stunden bei dieser Temperatur gehalten. Anschließend wird der Reaktionsansatz in bevorzugter Weise auf eine Temperatur von ca. 110 °C abgekühlt und während weiteren ca. 48 Stunden bei dieser Temperatur gehalten. Bevorzugt werden als Anfangskonzentrationen für die Aminosäuren Konzentrationen zwischen 2 und 20 mol/l, insbesondere zwischen 5 und 17 mol/l, vorzugsweise eine Konzentration von ca. 15 mol/l, verwendet.

Weiterhin kann es erfindungsgemäß bevorzugt sein, daß die Aminosäuren durch Silylierung, insbesondere durch Reaktion mit Hexamethyldisilazan (HMDS), aktiviert werden. Durch die Silylierung entstehen silylierte Aminosäuren, wobei die Heteroatome der Aminosäuren mindesten teilweise kovalent mit Silylgruppen, insbesondere mit Trimethylsilylgruppen, verbunden sind. Die auf diese Weise aktivierten Aminosäuren können zur Polyaminosäure polymerisiert werden.

Eine geeignete und bevorzugte Herstellungsmethode ist die fermentative Herstellung der Polyaminosäuren, insbesondere von Poly-Lysin, mit Hilfe von Bakterien. Auf diese Weise können sterisch reine Polyaminosäuren unter milden Bedingungen hergestellt werden.

Die hergestellten Polyaminosäuren können insbesondere durch Filtration und/oder Dialyse aufgereinigt werden. Bei kationischen, insbesondere polykationischen, Polyaminosäuren erfolgt die Aufreinigung mit besonderem Vorteil mit Hilfe der sogenannten CMC(Carboxymethylcellulose)-Methode. Bei der CMC-Methode bilden die Polyaminosäuren mit der in wäßrig-basischer Umgebung löslichen polyanionischen Carboxymethylcellulose vorzugsweise stabile unlösliche Aggregate. Diese Aggregate können durch Filtration von der wäßrigen Umgebung abgetrennt und insbesondere beliebig oft gewaschen werden. Somit können sämtliche Verunreinigungen beseitigt werden. Die Freisetzung der Polyaminosäuren aus den unlöslichen Aggregaten erfolgt vorzugsweise durch Ansäuern in wäßriger Umgebung, da auf diese Weise die protonierte Carboxymethylcellulose (CMC) unlöslich bleibt und die Polyaminosäuren in Lösung gehen. Gegebenenfalls kann bei Anwendung der CMC-Methode auf den Dialyseschritt zur Reinigung der Polyaminosäuren verzichtet werden. Durch geeignete Wahl der Parameter, insbesondere des pH-Wertes, der Konzentration, der Zugabegeschwindigkeit und der Art der verwendeten Carboxymethylcellulose, kann erreicht werden, daß einzelne Aminosäuren und Oligomere der Polyaminosäure keine Aggregate mit CMC bilden und mit Vorteil durch Waschschritte entfernt werden können. Vorzugsweise werden mit Hilfe der oben beschriebenen erfindungsgemäßen Verfahren verzweigte, insbesondere hyperverzweigte, Polyaminosäuren, insbesondere Homopolyaminosäuren, vorzugsweise Poly-s-Lysin, hergestellt. Bezüglich weiterer Einzelheiten wird auf die bisherige Beschreibung verwiesen.

Eine Übersicht verschiedener Herstellungsmöglichkeiten von Polyaminosäuren am Beispiel von Homo- und Heteropoly-Lysin ist in der nachfolgenden Tabelle aufgeführt.

Übersicht über die Polymerisationen von Lysin

| | **1.) Klassische Polykondensation** | **2.) Polymerisation mittels EDC** | **3.) Polymerisation mittels DCC/NHS** | **4.) Polymerisation mittels CDI** |
|---|---|---|---|---|
| **Eingesetztes Monomer** | Lysin oder Lysin*HCl/NaOH | Lysin*HCl | Lysin /Alanin | Lysin |
| **Reaktionsmedium** | Wasser | Wasser | Ethylacetat | Ethylacetat |
| **Reaktionstemperatur (-Zeit)** | 160 °C (2d)/120 °C (2) | RT (7 d) | RT (10 d) | RT (4 d) |
| **Aktivierungsreagenz [Äqu.]** | **-** | 1-Ethyl-3-(3-dimethylamino-propyl)carbodiimid [3.0] | Dicyclohexyl-carbodiimid [1.4], N-Hydroxy-succinimid [3.7] | Carbonyldiimidazol [2.6] |
| **Aufarbeitung** | Dialyse | Dialyse | Filtration, Soxhlet-Extraktion, Dialyse | Filtration, Dialyse |
| Monomereinheiten (ME) der synthetisierten Polymere | 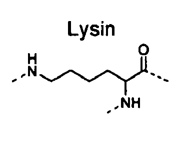 | 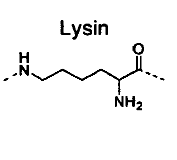 | 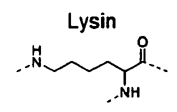 | 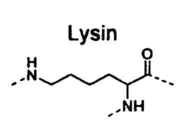 |
| | | | 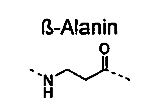 | |

Die Polyaminosäure wird in flüssiger Phase amphiphil modifiziert. Zur Herstellung der flüssigen Phase kommen insbesondere organische Lösungsmittel oder -gemische, insbesondere tertiäres Butanol in Betracht. Bevorzugt wird die Polyaminosäure in flüssiger Phase z.B. tertiäres Butanol suspendiert und erwärmt. Zu der Lösung bzw. Suspension der zu modifizierenden Polyaminosäure wird das für die amphiphile Modifikation vorgesehene Epoxid hinzugegeben. Die Modifizierungsreaktion wird vorzugsweise bei Temperaturen im Bereich von 30 bis 80°C insbesondere 40 bis 60°C durchgeführt und das erhaltene Produkt durch Dialyse aufgereinigt.

Weiterhin umfasst die Erfindung die Verwendung eines Komplexmaterials aus Metallnanopartikeln und Makromolekülen, wobei die Makromoleküle mindestens teilweise aus einer Polyaminosäure gebildet werden und insbesondere jeden Metallnanopartikel hüllenartig umgeben, als Biozid bei einem medizintechnischen Produkt. Bezüglich weiterer Merkmale und Einzelheiten wird auf die bisherige Beschreibung Bezug genommen.

Das erfindungsgemäße Produkt weist durch die molekulare Struktur und den Aufbau seiner Ausstattung bioverträgliche, insbesondere gewebeverträgliche, und gleichzeitig äußerst wirkungsvolle antimikrobielle bzw. biozide Eigenschaften auf. Die Zusammensetzung des Komplexmaterials aus körpereigenen Stoffen bzw. aus Stoffen, die aus körpereigenen Verbindungen aufgebaut sind, und/oder aus zumindest im wesentlichen körperverträglichen Stoffen gewährleistet die soeben angeführte Bioverträglichkeit des antimikrobiell ausgestatteten Produktes.

Die antimikrobiellen Eigenschaften der Ausstattung beruhen sowohl auf der bioziden Wirkung der Metallnanopartikel, insbesondere Silbernanopartikel, als auch auf der bioziden Wirkung der Polyaminosäure, insbesondere Poly-ε-Lysin. Die Zusammenführung dieser antimikrobiell wirkenden Stoffe in Form eines Komplexmaterials bedingt dessen hohe Wirksamkeit gegen insbesondere schädliche Mikroorganismen bzw. Keime. Mit besonderem Vorteil kann durch eine Core-Shell-Struktur des Komplexmaterials einerseits die Stabilisierung der Metallnanopartikel in unpolaren Lösungsmitteln bewirkt werden und damit eine Ausfällung und unkontrollierbare Akkumulation des Metalls verhindert werden. Andererseits vermittelt die hydrophobe Schale (Shell) der Core-Shell-Struktur die Haftung der Ausstattung insbesondere auf der Produktoberfläche. Auf diese Weise kann das Risiko einer unkontrollierbaren oder auch einer kontinuierlichen Freisetzung des Metalls in die Umgebung, insbesondere in das umliegende Körpergewebe, verringert werden. Somit können in diesem Zusammenhang möglicherweise auftretende Nebenwirkungen abgeschwächt oder im wesentlichen verhindert werden.

Weitere Merkmale der Erfindung ergeben sich durch die nachfolgende Beschreibung von bevorzugten Ausführungsformen anhand von Beispielen. Hierbei können die einzelnen Merkmale der Erfindung allein oder in Kombination miteinander verwirklicht sein. Die beschriebenen Ausführungsformen dienen lediglich zur Erläuterung und zum besseren Verständnis der Erfindung und sind in keiner Weise einschränkend zu verstehen.

### Beispiel 1: Herstellung von ε-Poly-L-Lysin

Zu einer Lösung aus 0.200 g L-Lysinhydrochlorid in 4 ml Wasser (dest.) wurden bei Raumtemperatur 0.627 g 1-Ethyl-3-[3-(dimethylamino)propyl] carbodiimidhydrochlorid in fünf Portionen zugegeben, wobei die Reaktionslösung zwischen zwei Zugaben 24 Stunden gerührt wurde. Nach Polymerisationsende wurde das Reaktionsgemisch gegen Wasser dialysiert (Dialysemembran mit einem Cut-off von 2000 g/mol) und anschließend das Polypeptid erhalten. (Ausbeute: 0,016 g [9 %]).

### Beispiel 2: Polymerisation von L-Lysin durch Zugabe von DCC/NHS

Zu einer gerührten farblosen Suspension aus 200 mg L-Lysin (1.37 mmol, 1.0 Äquivalente) in 5 ml Ethylacetat wurden 566 mg (2.74 mmol, 2.0 Äquivalente) Dicyclohexylcarbodiimid und 316 mg (2.74 mmol, 2.0 Äquivalente) N-Hydroxysuccinimid (NHS) zugegeben. Die milchige Suspension wurde 4 Tage bei Raumtemperatur gerührt und anschließend durch Entfernung des Lösungsmittels bis zur Trockene eingeengt. Der entstandene Feststoff wurde mit 20 ml Wasser versetzt und die entstandene farblose Suspension zentrifugiert (10 Minuten bei 4.000 Umdrehungen pro Minute). Der abgetrennte farblose Feststoff wurde zweimal mit je 10 ml Wasser gewaschen. Die vereinigten wässrigen klaren Phasen wurden auf ca. 10 ml eingeengt, wobei eine Trübung eintrat. Die entstandene Suspension wurde filtriert und das Filtrat gegen Wasser dialysiert (MWCO 2000). Aus der Dialyse wurden 55 mg von farblosem Poly-L-Lysin (0.43 mmol Lysin-Einheiten, Ausbeute: 33 %) erhalten.

### Beispiel 3: Alternative zu Beispiel 2

Zu einer gerührten farblosen Suspension aus 10.221g L-Lysin (0.070 mol, 1.0 Äqu.) in 180 ml Ethylacetat wurden 24.426g Dicyclohexylcarbodiimid (0.118 mol, 1.7 Äqu.) und 26.963g N-Hydroxy-succinimid (0.234 mol, 3.4 Äqu.) zugegeben. Die milchige Suspension wurde 10 Tage bei Raumtemperatur gerührt und anschließend durch Abziehen des Lösungsmittels im Vakuum bis zur Trockene eingeengt. Der entstandene Feststoff wurde mit 200 ml Wasser versetzt und die Suspension filtriert und mit je 100 und 200 ml Wasser gewaschen. Die vereinigten wässrigen Phasen wurden eingeengt, wobei eine Trübung eintrat, und anschließend gefriergetrocknet. Der wasserfreie Feststoff wurde anschlie-ßend fünf Tage durch Soxhlet-Extraktion in Ethanol gereinigt. Der Rückstand in der Soxhlet-Hülse wurde getrocknet und in Wasser gelöst. Die von der Hülse entfernten Reste wurden per Filtration beseitigt. Die wässrige Lösung wurde gegen Wasser dialysiert (MWCO 2000 g/mol), wobei das Lösungsmittel insgesamt siebenmal nach je 12 h erneuert wurde. Aus der Dialyse wurde nach Einengen 1.730g farbloses Polymer erhalten.

### Beispiel 4: Silylierung von L-Lysin mit Hexamethyldisilazan (HMDS)

Es wurden 9.14 g L-Lysinhydrochlorid (0.05 mol, 1.0 Äquivalente) in 53 ml Hexamethyldisilazan (41.02 g, 0.254 mmol, 5.1 Äquivalente) suspendiert und die Suspension auf 130 °C erhitzt. Nach 8 Stunden wurde eine gelbe Lösung erhalten. Nach 24 Stunden wurde die Lösung dunkelbraun. Es wurde restliches Hexamethyldisilazan im Vakuum (63 °C, 20 mbar) entfernt und eine dunkelbraune ölige Masse erhalten. Über Destillation (90 °C, 0.1 mbar) wurden 5.68 g farbloses Öl von silyliertem L-Lysin erhalten. Mit einem Silylierungsgrad von 1.9 pro Lysinmolekül berechnete sich eine Ausbeute von 40 % bezüglich des eingesetzten L-Lysins.

### Beispiel 5: Polymerisation des silylierten L-Lysins

Es wurden 2.860 mg des silylierten L-Lysins (ca. 0.012 mol Lysin-Anteil und ca. 0.023 mol, 1.0 Äquivalente, Trimethylsilyl-Anteil) unter Rückfluß auf 80 °C erhitzt und 1.0 ml Isopropanol (0.785 g, 0.013 mol, 0.6 Äquivalente) langsam dazugetropft. Es bildete sich langsam ein orangener Feststoff in einer hellgelben Flüssigkeit. Nach 8 Stunden wurde auf Raumtemperatur abgekühlt, und es wurden weitere 1.4 ml Isopropanol zugegeben. Nach einer Stunde wurden 2 ml Wasser zugegeben und das gesamte Lösungsmittel sowie das entstandene Hydroxytrimethylsilan im Vakuum entfernt. Der Rückstand wurde in Wasser gelöst und gegen Wasser dialysiert (MWCO 2000). Aus der Dialyse wurden 60 mg gelbliches Polymer (ca. 4 % Ausbeute) erhalten.

### Beispiel 6: Polykondensation

Es wurden 7.422 g L-Lysin *H₂O (0.045 mol) in 5.0 ml Wasser im Ultraschallbad gelöst. Anschließend wurde am Rotationsverdampfer so viel Wasser entfernt, bis eine Trübung eintrat, welche an Luft in eine klare dickflüssige Lösung überging. Durch Differenzwägung ergab sich, dass 3.0 ml Wasser als Lösungsmittel verblieben. Die wässrige L-Lysinlösung wurde auf 160 °C (Ölbadtemperatur) unter Rückfluss erhitzt und 2 Tage lang gerührt. Anschließend wurde auf 120 °C heruntertemperiert und weitere 2 Tage gerührt. Es wurde während der Reaktion regelmäßig ein leichter Stickstoffstrom für wenige Minuten durch die Apparatur geleitet, um Wasser zu entfernen. Nach beendeter Reaktion wurde auf Raumtemperatur abgekühlt und die sehr zähe orangene Masse in Wasser gelöst und anschließend gegen Wasser dialysiert (MWCO 2000). Aus der Dialyse wurden 2.278 g Polymer erhalten (Ausbeute: ca. 37 %).

### Beispiel 7: Vernetzung von ε-Poly-L-Lysin durch Zitronensäure

Es wurde zunächst eine Polymerisation von 0.200 g L-Lysinhydrochlorid (1.00 Äquivalente) gemäß Beispiel 1 durchgeführt. Anschließend wurde nach 24 Stunden der letzten EDC-Zugabe eine frisch zubereitete Lösung aus 0.011 g Zitronensäure (0.055 mmol, 0.05 Äquivalente) und 0.032 g EDC-HCL (0.167 mmol, 0.15 Äquivalente) in 1 ml Wasser zugegeben. Für eine höhere Vernetzung wurden bei gleicher Vorgehensweise und gleichem Ansatz 0.021 g Zitronensäure (0.110 mmol, 0.10 Äquivalente) und 0.064 g EDC·HCL (0.334 mmol, 0.30 Äquivalente) eingesetzt.

### Beispiel 8: Modifizierung von ε-Poly-L-Lysin

501.5 mg (3.912 mmol ME, 1.00 Äqu.) Poly-ε-Lysin (PL) wurden in 30 ml tert-Butanol suspendiert und die Suspension bei 50 °C unter Rückfluss gerührt. Nach 15 Minuten wurden 1516.0 mg (5.079 mmol, 1.31 Äqu.) Glycidylhexadecylether (GHE) zugegeben. Die Suspension wurde für weitere 20 Stunden bei 50 °C gerührt. Nach Abziehen des Lösungsmittels am Rotationsverdampfer wurde das trockene Rohprodukt in 50 ml Isopropanol bei 40 °C gelöst und anschließend gegen Isopropanol dialysiert (MWCO 2000 g/mol). Aus der Dialyse wurden nach Abzug des Lösungsmittels 1067.3 mg des hydrophob modifizierten GHE-PL erhalten. Bei dem durch GHE hydrophobmodifizierte PL wurde durch den TNBS-Test, bei dem quantitativ auf Aminogruppen getestet wird, wurde ein Modifizierungsgrad von 73 % der freien Aminogruppen des PL ermittelt.

Unter Verwendung von 0,43 bzw. 0,83 Äquivalenten GHE werden Modifizierungsgrade von 10 bzw. 47 % erhalten.

### Beispiel 9: Vernetzung und Modifizierung von fermentativ hergestelltem Poly-Lysin

Zu einer Lösung aus 500.3 mg ε-Lysin (3.903 mmol ME, -NH2, 1.00 Äqu., Chisso Corp., Japan, Lot. D3050804) in 10 ml deionisiertem Wasser wurde eine frisch zubereitete Lösung aus 38.4 mg Zitronensäure (0.201 mmol, 0.05 Äqu.) und 116.1 mg EDC*HC1 (0.604 mmol, 0.15 Äqu.) in 3 ml deion. Wasser gegeben. Die Mischung wurde anschließend bei Raumtemperatur für 24 h gerührt und dann eingeengt und lyophilisiert.
Das Rohprodukt (ca. 3.32 mmol -NH2, 1.00 Äqu.) wurde in 30 ml tertiäres Butanol suspendiert und die Suspension bei 50 °C unter Rückfluß gekocht. Nach 15 Minuten wurden 1301.1 mg (4.359 mmol, ca. 1.31 Äqu.) Glycidylhexadecylether zugegeben und die Suspension wurde für weitere 20 h bei 50 °C gerührt. Nach Abziehen des Lösungsmittels wurde das trockene Rohprodukt in 50 ml iso-Propanol bei 40 °C aufgelöst und die Lösung anschließend gegen iso-Propanol dialysiert (MWCO 2000 g/mol). Aus der Dialyse wurden nach Abzug des Lösungsmittels das gewünschte Polymer (zitronensäure-vernetztes und GEHEmodifiziertes e-Poly-Lysin) erhalten.

### Beispiel 10: Beladen der Polymerartikel mit Silber(I)-nitrat und Reduktion

Es wurden unter Stickstoffatmosphäre 10 mg modifiziertes ε-Poly-L-Lysin in 7,6 ml Toluol gelöst und 5,7 mg AgNO₃ in drei Portionen (24 Stunden Rührzeit nach jeder Zugabe) zugegeben. Es wurde eine klare und stabile Silber(1)-Polymer-Toluol-Lösung erhalten. Es wurden 0,1 ml der Silber(1)-Polymer-Toluol-Lösung (Inhalt: 0,13 mg Polymer, 0,05 mg AgNO₃) mit 2 ml i-Propanol verdünnt und mit 0,03 ml einer 0,02 M L-Ascorbinsäure-Lösung (in i-Propanol) versetzt. Die Lösung färbte sich intensiv gelb. Alternativ können 0,1 ml der Silber(1)-Polymer-ToluolLösung (Inhalt: 0,13 mg Polymer, 0,05 mg AgNO₃) mit 2 ml i-Propano) verdünnt und mit 0,06 ml einer verdünnten 0,01 M (M: Molarität) Li[HBEt₃]-Lösung (in THF) versetzt werden. Die Lösung färbt sich ebenfalls intensiv gelb.

### Beispiel 11: Beladen der Polymerartikel mit Silber(1)-nitrat ohne Reduktionsmittel

Das GHE-PL von Beispiel 8 wurde unter Argonatmosphäre in Toluol gelöst und portionsweise Silbernitrat (AgNO₃) zugefügt. Dabei konnten in 50.0 mg GHE-PL 19.03 mg Silbernitrat gelöst werden (entspricht 38.1 Gew.%). Die Aufnahmefähigkeit von Silber wurde in diesem Fall um das Sechsfache erhöht im Vergleich zu einer Modifizierung mit Fettsäuren. Ein weiterer Vorteil der GHE-Modifizierung besteht in den selbstreduzierenden Eigenschaften. Bei der Beladung von GHE-PL mit Silbernitrat tritt nach einiger Zeit die gelb bis braune Färbung der Silber-Nanopartikel auf. Silbernitrat kann in GHE-PL also ohne Zusatz von Reduktionsmittel reduziert werden. Die Reduzierung des ionischen Silbers zu neutralem Silber erfolgt unter gleichzeitiger Oxidation der aus der Epoxigruppe gebildeten, der sekundären Aminogruppe benachbarten Hydroxylgruppe zur Ketogruppe. Diese Selbstreduktion kann durch Wärmezufuhr (1 h bei 90 °C) weiter unterstützt werden. Die Reduktion des Silbernitrats kann wenn erwünscht auch durch Zusatz von Reduktionsmittel wie zum Beispiel Ascorbinsäure beschleunigt werden.

### Beispiel 12: Herstellung von Filmen auf Glasobjektträgern

Zur Herstellung eines Films wurde eine Silber(0)-Polymer-Lösung auf eine markierte Fläche von ca. 1 cm² eines als Modell für ein medizinisches Produkt dienenden Glasobjektträgers mit einer Pipette aufgetragen. Der Objektträger wurde auf eine Heizplatte gelegt und das Lösungsmittel abgedampft. Dabei wurde mit der Pipette die immer konzentrierter werdende Lösung auf dem Glas verrührt, so dass ein Film in der markierten Zone gebildet wurde.
Der Komplex aus Metallnanopartikeln und modifizierter Polyaminosäure kann als solcher oder zusammen mit dem medizinischen Produkt sterilisiert werden, z.B. hitzesterilisiert (4 h bei 180 °C).

### Beispiel 13: Bakterientests

Es wurden für die Bakterientests ca. 1 cm² große Filme auf Glasobjektträgern hergestellt: 4 µg Silber in 40 µg Polymer bzw. 10 µg Silber in 100 µg Polymer. Zur Vorbereitung der Bakterienzellen wurden 50 ml eines sterilen Standard-Kulturmediums der Fa. Merck mit 100 µl Suspension von Staphylokokkus Aureus-Zellen (ca. 10¹¹ Zellen pro ml) in PBS (Phosphate buffered saline, pH 7.0) angeimpft und für 6 Stunden bei 37 °C unter Schütteln inkubiert. Nach Zentrifugieren der Bakteriensuspension wurden die Zellen zweimal mit PBS (pH 7.0) gewaschen, dann mit PBS resuspendiert und weiter mit PBS auf eine Konzentration von 5 x 10⁸ Zellen pro ml verdünnt. Die Zellkonzentration wurde durch die Absorption bei 600 nm überprüft. Die hergestellten Filme wurden für 2 Minuten in PBS gewaschen und mit der Bakteriensuspension besprüht. Anschließend wurden die Objektträger in je eine Petri-Schale gelegt und 25 ml an Wachstumsagar (1.5 Gew.% Agar in Wachstumsmedium wurde für 5 Minuten auf 100 °C erhitzt und schnell auf 40 °C abgekühlt) zugegeben. Danach wurden die Petri-Schalen bei 37 °C inkubiert. Die Filme mit einer Mindestmenge an Silber von 10 µg pro cm² verhinderten das Wachstum der aufgesprühten Staphylokokkus Aureus-Zellen zu mehr als 99 %.

## Patentansprüche

1. Medizintechnisches Produkt mit einer antimikrobiellen Ausstattung aus einem Komplexmaterial aus Metallnanopartikeln und Makromolekülen, wobei die Makromoleküle mindestens teilweise aus amphiphil modifizierten Polyaminosäuren gebildet werden, die Aminosäurebausteine enthalten, die außer den in die Peptidbindung eingebundenen funktionellen Gruppen mindestens eine weitere funktionelle Gruppe aufweisen und mindestens ein Teil dieser Gruppen mit hydrophobe Reste tragenden Substanzen über kovalente Bindungen modifiziert sind, **dadurch gekennzeichnet, dass** die hydrophoben Reste durch Reaktion von mindestens eine Epoxy-Gruppen tragenden Substanzen mit der mindestens einen weiteren funktionellen Gruppe und/oder mit mindestens einer Aminogruppe eines mindestens eine Aminogruppe als weitere funktionelle Gruppe tragenden Aminosäurebausteins unter Aufrechterhaltung einer Aminofunktion an die Polyaminosäuren gebunden sind.

2. Medizintechnisches Produkt nach Anspruch 1, **dadurch gekennzeichnet, dass** die Ausstattung zumindest auf einem Teil der Oberfläche des Produktes, insbesondere in Form einer Beschichtung, vorgesehen ist.

3. Medizintechnisches Produkt nach Anspruch 1 oder 2, **dadurch gekennzeichnet, dass** die Ausstattung im Inneren des Produktes vorgesehen ist.

4. Medizintechnisches Produkt nach einem der vorhergehenden Ansprüche, **dadurch gekennzeichnet, dass** jeder Metallnanopartikel hüllenartig von mindestens einer modifizierten Polyaminosäure umgeben ist.

5. Medizintechnisches Produkt nach einem der vorhergehenden Ansprüche, **dadurch gekennzeichnet, dass** die Polyaminosäuren Homo- und/oder Heteropolyaminosäuren, insbesondere Homopolyaminosäuren, sind.

6. Medizintechnisches Produkt nach einem der vorhergehenden Ansprüche, **dadurch gekennzeichnet, dass** die Polyaminosäuren aus natürlich vorkommenden und/oder synthetischen, insbesondere aus natürlich vorkommenden, Aminosäurebausteinen bestehen.

7. Medizintechnisches Produkt nach einem der vorhergehenden Ansprüche, **dadurch gekennzeichnet, dass** die Polyaminosäuren mindestens einen basischen, sauren und/oder schwefelhaltigen Aminosäurebaustein, insbesondere mindestens eine Aminosäure aus der Gruppe, umfassend Cystein, Methionin, Tryptophan, Histidin, Arginin, Lysin, Ornithin, Asparaginsäure, Glutaminsäure und deren Derivate, enthalten, wobei vorzugsweise mindestens 50
% der Aminosäurebausteine einer Polyaminosäure aus solchen Aminosäurebausteinen gebildet sind.

8. Medizintechnisches Produkt nach einem der vorhergehenden Ansprüche, **dadurch gekennzeichnet, dass** die Polyaminosäuren eine lineare Struktur aufweisen.

9. Medizintechnisches Produkt nach einem der vorhergehenden Ansprüche, **dadurch gekennzeichnet, dass** Polyaminosäuren verzweigte, inbesondere stark verzweigte und/oder vernetzte, Polyaminosäuren sind.

10. Medizintechnisches Produkt nach einem der vorhergehenden Ansprüche, **dadurch gekennzeichnet, dass** die Polyaminosäuren ausschließlich aus Aminosäuren mit mindestens einer weiteren funktionellen Gruppe gebildet sind.

11. Medizintechnisches Produkt nach einem der vorhergehenden Ansprüche, **dadurch gekennzeichnet, dass** die noch nicht modifizierten Polyaminosäuren ausschließlich aus Aminosäurebausteinen bestehen, bei denen die mindestens eine weitere funktionelle Gruppe eine nukleophile Gruppe, insbesondere eine Aminogruppe, ist und wobei die Aminogruppe vorzugsweise eine primäre Aminogruppe ist, die durch Anlagerung des hydrophoben Restes in eine sekundäre Aminogruppe umgewandelt ist.

12. Medizintechnisches Produkt nach einem der vorhergehenden Ansprüche, **dadurch gekennzeichnet, dass** die Polyaminosäure Polylysin, insbesondere Poly-α-Lysin und/oder Poly-ε-Lysin, vorzugsweise Poly-ε-Lysin, ist.

13. Medizintechnisches Produkt nach einem der vorhergehenden Ansprüche, **dadurch gekennzeichnet, dass** die noch nicht modifizierte Polyaminosäure ein Molekulargewicht im Bereich von 500 bis 1.000.000 g/mol, insbesondere 3.000 bis 100.000 g/mol besitzt.

14. Medizintechnisches Produkt nach einem der vorhergehenden Ansprüche, **dadurch gekennzeichnet, dass** die Polyaminosäure mit mindestens einem Glycidylether mit mindestens einem aliphatischen Rest amphiphil modifiziert ist.

15. Medizintechnisches Produkt nach einem der vorhergehenden Ansprüche, **dadurch gekennzeichnet, dass** der hydrophobe Rest ein linearer aliphatischer Rest mit einer vorzugsweise geraden Zahl an Kohlenstoffatomen ist.

16. Medizintechnisches Produkt nach einem der vorhergehenden Ansprüche, **dadurch gekennzeichnet, dass** der hydrophobe, insbesondere aliphatische, Rest 8 bis 24, insbesondere 12 bis 20, vorzugsweise 16 und/oder 18 Kohlenstoffatome aufweist.

17. Medizintechnisches Produkt nach einem der Ansprüche 14 bis 16, **dadurch gekennzeichnet, dass** der Glycidylether Glycidylhexadecylether und/oder Glycidyloctadecylether ist.

18. Medizintechnisches Produkt nach einem der vorhergehenden Ansprüche, **dadurch gekennzeichnet, dass** die noch nicht modifizierte Polyaminosäure eine globuläre Struktur aufweist, insbesondere verzweigt und/oder vernetzt ist.

19. Medizintechnisches Produkt nach einem der vorhergehenden Ansprüche, **dadurch gekennzeichnet, dass** die Polyaminosäuren mit einer vernetzenden Komponente, insbesondere mit einem polyfunktionellen Epoxid oder einer polyfunktionellen Carbonsäure, vernetzt sind.

20. Medizintechnisches Produkt nach einem der vorhergehenden Ansprüche, **dadurch gekennzeichnet, dass** es sich bei den Metallnanopartikeln um Gold-, Silber-, Kupfer oder Zinknanopartikel handelt, wobei Silbernanopartikel bevorzugt sind.

21. Medizintechnisches Produkt nach einem der vorhergehenden Ansprüche, **dadurch gekennzeichnet, dass** die Metallnanopartikel einen Durchmesser von 0,5 bis 20 nm, insbesondere 1 bis 20 nm, vorzugsweise 1 bis 14 nm, aufweisen.

22. Verfahren zur Herstellung des medizintechnischen Produktes nach einem der Ansprüche 1 bis 21, **dadurch gekennzeichnet, dass** die antimikrobielle Ausstattung, insbesondere in Form einer Lösung, von außen auf das nicht ausgestattete Produkt aufgebracht und/oder in den Werkstoff des Produktes bei dessen Herstellung eingebracht wird.

23. Verfahren zur Herstellung von mindestens einer Polyaminosäure, insbesondere zur Herstellung des medizintechnischen Produktes nach einem der Ansprüche 1 bis 21, durch Polymerisation von Aminosäuren, die mindestens zum Teil mindestens trifunktionelle Aminosäuren sind, in flüssiger Phase, **dadurch gekennzeichnet, dass** die Aminosäuren ohne Verwendung von Schutzgruppen polymerisiert werden und die Polyaminosäure in flüssiger Phase amphiphil modifiziert wird, wobei zu einer Lösung bzw. Suspension der zu modifzierenden Polyaminosäure ein für die amphiphile Modifikation vorgesehenes Epoxid hinzugegeben wird.

24. Verfahren nach Anspruch 23, **dadurch gekennzeichnet, dass** die Polymerisation der Aminosäuren bei Temperaturen im Bereich von 80 bis 200 °C, insbesondere bei 100 bis 180 °C, vorzugsweise 140 bis 160 °C, durchgeführt wird.

25. Verfahren nach Anspruch 23, **dadurch gekennzeichnet, dass** die Aminosäuren für die Polymerisation aktiviert werden.

26. Verfahren nach einem der Ansprüche 23 bis 25, **dadurch gekennzeichnet, dass** die Polyaminosäuren, insbesondere lineare Polyaminosäuren, vernetzt werden, insbesondere vor der Modifizierung.

27. Komplexmaterial aus Metallnanopartikeln und Makromolekülen, wobei die Makromoleküle mindestens teilweise aus amphiphil modifizierten Polyaminosäuren gebildet werden, die außer den in die Peptidbindung eingebundenen Gruppen mindestens eine weitere funktionelle Gruppe aufweisen und mindestens ein Teil dieser weiteren funktionellen Gruppen mit hydrophobe Reste tragenden Substanzen über kovalente Bindungen modifiziert ist, **dadurch gekennzeichnet, dass** die hydrophoben Reste durch Anlagerung von Epoxy-Gruppen tragenden Substanzen an die mindestens eine weitere funktionelle Gruppe und/oder über mindestens eine Aminogruppe eines mindestens eine Aminogruppe als weitere funktionelle Gruppe tragenden Aminosäurebausteins unter Aufrechterhaltung einer Aminofunktion an die Polyaminosäuren gebunden sind.

28. Verwendung des Komplexmaterials nach Anspruch 27 als Biozid, insbesondere bei einem medizintechnischen Produkt.

## Claims

1. A medicotechnical product having an antimicrobial finish consisting of a complex material of metal nanoparticles and macromolecules, the macro-molecules being at least partially formed from amphiphilically modified polyamino acids which contain amino acid units, which aside from the functional groups bonded in the peptide bond contain at least one further functional group and at least some of these groups are modified with substances bearing hydrophobic radicals via covalent bonds, **characterized in that** the hydrophobic radicals are bonded by reaction of substances bearing at least one epoxy group with the at least one further functional group and/or with at least one amino group of an amino acid unit bearing at least one amino group as a further functional group with maintenance of an amino function on the polyamino acids.

2. The medicotechnical product as claimed in claim 1, **characterized in that** the finish is provided at least on one part of the surface of the product, in particular in the form of a coating.

3. The medicotechnical product as claimed in claim 1 or 2, **characterized in that** the finish is provided in the interior of the product.

4. The medicotechnical product as claimed in any of the preceding claims, **characterized in that** each metal nanoparticle is surrounded in a shell-like manner by at least one modified poly-amino acid.

5. The medicotechnical product as claimed in any of the preceding claims, **characterized in that** the polyamino acids are homo- and/or hetero-polyamino acids, in particular homopolyamino acids.

6. The medicotechnical product as claimed in any of the preceding claims, **characterized in that** the polyamino acids consist of naturally occurring and/or synthetic, in particular of naturally occurring, amino acid units.

7. The medicotechnical product as claimed in any of the preceding claims, **characterized in that** the polyamino acids contain at least one basic, acidic and/or sulfur-containing amino acid unit, in particular at least one amino acid from the group comprising cysteine, methionine, tryptophan, histidine, arginine, lysine, ornithine, aspartic acid, glutamic acid and their derivatives, and preferably at least 50% of the amino acid units of a polyamino acid being formed from amino acid units of this type.

8. The medicotechnical product as claimed in any of the preceding claims, **characterized in that** the polyamino acids have a linear structure.

9. The medicotechnical product as claimed in any of the preceding claims, **characterized in that** the polyamino acids are branched, in particular highly branched and/or crosslinked, polyamino acids.

10. The medicotechnical product as claimed in any of the preceding claims, **characterized in that** the polyamino acids are exclusively formed from amino acids having at least one further functional group.

11. The medicotechnical product as claimed in any of the preceding claims, **characterized in that** the still unmodified polyamino acids consisting exclusively of amino acid units in which the at least one further functional group is a nucleophilic group, in particular an amino group, and the amino group preferably being a primary amino group which is converted into a secondary amino group by addition of the hydrophobic radical.

12. The medicotechnical product as claimed in any of the preceding claims, **characterized in that** the polyamino acid is polylysine, in particular poly-α-lysine and/or poly-ε-lysine, preferably poly-ε-lysine.

13. The medicotechnical product as claimed in any of the preceding claims, **characterized in that** the still unmodified polyamino acid has a molecular weight in the range from 500 to 1 000 000 g/mol, in particular 3000 to 100 000 g/mol.

14. The medicotechnical product as claimed in any of the preceding claims, **characterized in that** the polyamino acid is amphiphilically modified by at least one glycidyl ether having at least one aliphatic radical.

15. The medicotechnical product as claimed in any of the preceding claims, **characterized in that** the hydrophobic radical is a linear aliphatic radical having a preferably even number of carbon atoms.

16. The medicotechnical product as claimed in any of the preceding claims, **characterized in that** the hydrophobic, in particular aliphatic, radical has 8 to 24, in particular 12 to 20, preferably 16 and/or 18, carbon atoms.

17. The medicotechnical product as claimed in any of the claims 14 through 16, **characterized in that** the glycidyl ether is glycidyl hexadecyl ether and/or glycidyl octadecyl ether.

18. The medicotechnical product as claimed in any of the preceding claims, **characterized in that** the still unmodified polyamino acid has a globular structure, in particular is branched and/or crosslinked.

19. The medicotechnical product as claimed in any of the preceding claims, **characterized in that** the polyamino acids are crosslinked with a crosslinking component, in particular with a poly-functional epoxide or a polyfunctional carboxylic acid.

20. The medicotechnical product as claimed in any of the preceding claims, **characterized in that** the metal nanoparticles are gold, silver, copper or zinc nanoparticles, silver nanoparticles being preferred.

21. The medicotechnical product as claimed in any of the preceding claims, **characterized in that** the metal nanoparticles have a diameter of 0.5 to 20 nm, in particular 1 to 20 nm, preferably 1 to 14 nm.

22. A method for the production of the medico-technical product as claimed in any of the claims 1 through 21, **characterized in that** the antimicrobial finish is applied, in particular in the form of a solution, to the unfinished product from outside and/or is introduced into the material of the product during its production.

23. A method for the production of at least one polyamino acid, in particular for the production of the medicotechnical product as claimed in any of the claims 1 through 21, by polymerization of amino acids which are at least partially at least trifunctional amino acids, in the liquid phase, **characterized in that** the amino acids are polymerized without use of protective groups and the polyamino acid is amphiphilically modified in the liquid phase, an epoxide is added to the solution or suspension of the polyamino acid to be modified.

24. The method as claimed in claim 23, **characterized in that** the polymerization of the amino acids is carried out at temperatures in the range from 80 to 200°C, in particular at 100 to 180°C, preferably 140 to 160°C.

25. The method as claimed in claim 23, **characterized in that** the amino acids are activated for the polymerization.

26. The method as claimed in any of claims 23 through 25, **characterized in that** the polyamino acids, in particular linear polyamino acids, are crosslinked, in particular before modification.

27. A complex material consisting of metal nanoparticles and macromolecules, the macro-molecules being at least partially formed from amphiphilically modified polyamino acids, which aside from the groups bonded in the peptide bond contain at least one further functional group and at least some of these further functional groups are modified by means of covalent bonds with substances bearing hydrophobic radicals, **characterized in that** the hydrophobic radicals are bonded by addition of substances bearing epoxy groups to the at least one further functional group and/or by means of at least one amino group of an amino acid unit bearing at least one amino group as a further functional group with maintenance of an amino function on the polyamino acids.

28. The use of the complex material as claimed in claim 27 as a biocide, in particular in a medicotechnical product.

## Revendications

1. Produit médico-technique présentant un apprêt antimicrobien constitué par un matériau complexe de nanoparticules métalliques et de macromolécules, les macromolécules étant formées au moins partiellement de poly(acides aminés) modifiés de manière amphiphile, qui contiennent des éléments d'acides aminés, présentant, outre les groupes fonctionnels intégrés dans la liaison peptidique, au moins un autre groupe fonctionnel et au moins une partie de ces groupes étant modifiés par des substances portant des radicaux hydrophobes via des liaisons covalentes, **caractérisé en ce que** les radicaux hydrophobes sont liés aux poly(acides aminés) par réaction de substances portant au moins un groupe époxy avec ledit au moins un autre groupe fonctionnel et/ou avec au moins un groupe amino d'un élément d'acide aminé portant au moins un groupe amino comme autre groupe fonctionnel avec conservation d'une fonction amino.

2. Produit médico-technique selon la revendication 1, **caractérisé en ce que** l'apprêt est prévu au moins sur une partie de la surface du produit, en particulier sous forme d'un revêtement.

3. Produit médico-technique selon la revendication 1 ou 2, **caractérisé en ce que** l'apprêt est prévu à l'intérieur du produit.

4. Produit médico-technique selon l'une quelconque des revendications précédentes, **caractérisé en ce que** chaque nanoparticule métallique est entourée par au moins un poly(acide aminé) modifié, sous forme d'une enveloppe.

5. Produit médico-technique selon l'une quelconque des revendications précédentes, **caractérisé en ce que** les poly(acides aminés) sont des homopoly(acides aminés) et/ou des hétéropoly(acides aminés), en particulier des homopoly(acides aminés).

6. Produit médico-technique selon l'une quelconque des revendications précédentes, **caractérisé en ce que** les poly(acides aminés) sont constitués par des éléments d'acides aminés naturels et/ou synthétiques, en particulier des éléments d'acides aminés naturels.

7. Produit médico-technique selon l'une quelconque des revendications précédentes, **caractérisé en ce que** les poly(acides aminés) contiennent au moins un élément d'acide aminé basique, acide et/ou soufré, en particulier au moins un acide aminé du groupe comprenant la cystéine, la méthionine, le tryptophane, l'histidine, l'arginine, la lysine, l'ornithine, l'acide aspartique, l'acide glutamique et leurs dérivés, de préférence au moins 50% des éléments d'acides aminés d'un poly(acide aminé) étant formés à partir de ces éléments d'acides aminés.

8. Produit médico-technique selon l'une quelconque des revendications précédentes, **caractérisé en ce que** les poly(acides aminés) présentent une structure linéaire.

9. Produit médico-technique selon l'une quelconque des revendications précédentes, **caractérisé en ce que** les poly(acides aminés) sont des poly(acides aminés) ramifiés, en particulier fortement ramifiés et/ou réticulés.

10. Produit médico-technique selon l'une quelconque des revendications précédentes, **caractérisé en ce que** les poly(acides aminés) sont formés exclusivement d'acides aminés présentant au moins un autre groupe fonctionnel.

11. Produit médico-technique selon l'une quelconque des revendications précédentes, **caractérisé en ce que** les poly(acides aminés) non encore modifiés sont exclusivement formés d'éléments d'acides aminés dans lesquels ledit au moins un autre groupe fonctionnel est un groupe nucléophile, en particulier un groupe amino et le groupe amino étant de préférence un groupe amino primaire qui est transformé par addition du radical hydrophobe en groupe amino secondaire.

12. Produit médico-technique selon l'une quelconque des revendications précédentes, **caractérisé en ce que** le poly(acide aminé) est la polylysine, en particulier la poly-α-lysine et/ou la poly-ε-lysine, de préférence la poly-ε-lysine.

13. Produit médico-technique selon l'une quelconque des revendications précédentes, **caractérisé en ce que** le poly(acide aminé) non encore modifié présente un poids moléculaire dans la plage de 500 à 1 000 000 g/mole, en particulier de 3000 à 100 000 g/mole.

14. Produit médico-technique selon l'une quelconque des revendications précédentes, **caractérisé en ce que** le poly(acide aminé) est modifié de manière amphiphile avec au moins un glycidyléther présentant au moins un radical aliphatique.

15. Produit médico-technique selon l'une quelconque des revendications précédentes, **caractérisé en ce que** le radical hydrophobe est un radical aliphatique linéaire présentant de préférence un nombre pair d'atomes de carbone.

16. Produit médico-technique selon l'une quelconque des revendications précédentes, **caractérisé en ce que** le radical hydrophobe, en particulier aliphatique, présente 8 à 24, en particulier 12 à 20, de préférence 16 et/ou 18 atomes de carbone.

17. Produit médico-technique selon l'une quelconque des revendications 14 à 16, **caractérisé en ce que** le glycidyléther est le glycidylhexadécyléther et/ou le glycidyloctadécyléther.

18. Produit médico-technique selon l'une quelconque des revendications précédentes, **caractérisé en ce que** le poly(acide aminé) non encore modifié présente une structure globulaire et est en particulier ramifié et/ou réticulé.

19. Produit médico-technique selon l'une quelconque des revendications précédentes, **caractérisé en ce que** les poly(acides aminés) sont réticulés avec un composant réticulant, en particulier avec un époxyde polyfonctionnel ou un acide carboxylique polyfonctionnel.

20. Produit médico-technique selon l'une quelconque des revendications précédentes, **caractérisé en ce qu'**il s'agit, pour les nanoparticules métalliques, de nanoparticules d'or, d'argent, de cuivre ou de zinc, les nanoparticules d'argent étant préférées.

21. Produit médico-technique selon l'une quelconque des revendications précédentes, **caractérisé en ce que** les nanoparticules métalliques présentent un diamètre de 0,5 à 20 nm, en particulier de 1 à 20 nm et de préférence de 1 à 14 nm.

22. Procédé pour la préparation du produit médico-technique selon l'une quelconque des revendications 1 à 21, **caractérisé en ce que** l'apprêt antimicrobien, en particulier sous forme d'une solution, est appliqué à partir de l'extérieur sur le produit non apprêté et/ou est incorporé dans le matériau du produit lors de sa fabrication.

23. Procédé pour la préparation d'au moins un poly(acide aminé), en particulier pour la fabrication du produit médico-technique selon l'une quelconque des revendications 1 à 21, par polymérisation d'acides aminés qui sont au moins en partie des acides aminés au moins trifonctionnels, en phase liquide, **caractérisé en ce que** les acides aminés sont polymérisés sans utilisation de groupes de protection et le poly(acide aminé) est modifié de manière amphiphile en phase liquide, en ajoutant à une solution ou une suspension du poly(acide aminé) à modifier un époxyde prévu pour la modification amphiphile.

24. Procédé selon la revendication 23, **caractérisé en ce que** la polymérisation des acides aminés est réalisée à des températures dans la plage de 80 à 200°C, en particulier de 100 à 180°C, de préférence de 140 à 160°C.

25. Procédé selon la revendication 23, **caractérisé en ce que** les acides aminés sont activés pour la polymérisation.

26. Procédé selon l'une quelconque des revendications 23 à 25, **caractérisé en ce que** les poly(acides aminés), en particulier des poly(acides aminés) linéaires sont réticulés, en particulier avant la modification.

27. Matériau complexe de nanoparticules métalliques et de macromolécules, les macromolécules étant formées au moins partiellement de poly(acides aminés) modifiés de manière amphiphile, qui présentent, outre les groupes fonctionnels intégrés dans la liaison peptidique, au moins un autre groupe fonctionnel et au moins une partie de ces autres groupes fonctionnels étant modifiée par des substances portant des radicaux hydrophobes via des liaisons covalentes, **caractérisé en ce que** les radicaux hydrophobes sont liés aux poly(acides aminés) par addition de substances portant des groupes époxy sur ledit au moins un autre groupe fonctionnel et/ou via au moins un groupe amino d'un élément d'acide aminé portant au moins un groupe amino comme autre groupe fonctionnel avec conservation d'une fonction amino.

28. Utilisation du matériau complexe selon la revendication 27 comme biocide, en particulier dans le cas d'un produit médico-technique.
